(19) Europäisches Patentamt
European Patent Office
Office européen des brevets

(11) Veröffentlichungsnummer: **0 225 866 B1**

# (12) EUROPÄISCHE PATENTSCHRIFT

(45) Veröffentlichungstag der Patentschrift: **25.09.91**

(51) Int. Cl.⁵: **C07D 239/95**, C07D 403/12, C07D 401/14, A61K 31/505

(21) Anmeldenummer: **86890328.7**

(22) Anmeldetag: **24.11.86**

Die Akte enthält technische Angaben, die nach dem Eingang der Anmeldung eingereicht wurden und die nicht in dieser Patentschrift enthalten sind.

## (54) Chinazolin-Derivate, Verfahren zu ihrer Herstellung, sie enthaltende pharmazeutische Präparate und ihre Verwendung.

(30) Priorität: **04.12.85 AT 3522/85**

(43) Veröffentlichungstag der Anmeldung: **16.06.87 Patentblatt 87/25**

(45) Bekanntmachung des Hinweises auf die Patenterteilung: **25.09.91 Patentblatt 91/39**

(84) Benannte Vertragsstaaten: **BE CH DE ES FR GB GR IT LI LU NL SE**

(56) Entgegenhaltungen: **DE-A- 3 419 223**

(73) Patentinhaber: **Gerot-Pharmazeutika Gesellschaft m.b.H.
Arnethgasse 3
A-1171 Wien(AT)**

(72) Erfinder: **Schlager, Ludwig H., Dr.
Nottebohmstrasse 12
A-1190 Wien(AT)**

(74) Vertreter: **Pawloy, Heinrich, Dr. et al
Riemergasse 14
A-1010 Wien(AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 225 866 B1

**Beschreibung**

Die Erfindung betrifft neue Chinazolin-Derivate der allgemeinen Formel (I)

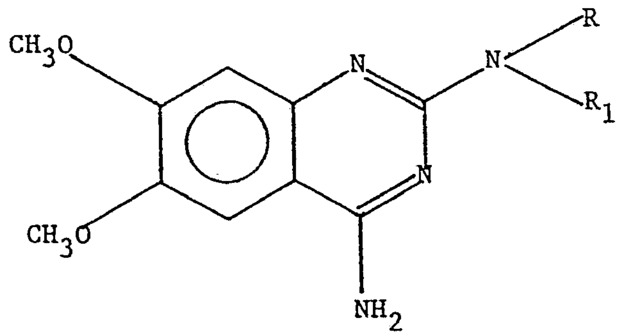

(I) ,

worin entweder R Wasserstoff und $R_1$ einen 3-(2-Pyrrolidinon-1-yl)-propyl-Rest darstellt oder worin R Methyl und $R_1$ einen 2-(Dimethoxyphenyl)-äthyl-Rest bedeutet oder worin R zusammen mit $R_1$ einen Piperidinring bildet, in dessen 4-Stellung ein 2-Keto-1-benzimidazolinyl-Rest gebunden ist oder worin R zusammen mit $R_1$ einen Piperazinring bildet, in dessen 4-Stellung ein Hydroxyacylrest mit höchstens 8 Kohlenstoffatomen gebunden ist.

Es gibt bereits eine Reihe von blutdrucksenkenden Chinazolin-Derivaten, die zwar der allgemeinen Formel (I) entsprechen, deren Reste R und $R_1$ jedoch eine andere Bedeutung haben. Einige dieser Wirkstoffe werden therapeutisch als $\alpha$-Blocker verwendet.

Die erfindungsgemäßen Chinazolin-Derivate der allgemeinen Formel (I) sind ebenfalls antihypertensive Wirkstoffe, von denen sich manche aber gegenüber dem Stand der Technik dadurch auszeichnen, daß sie sowohl $\alpha$- als auch $\beta$-adrenolytische Wirkung zeigen, schon als Basen wasserlöslich sind und eine besonders geringe Toxizität sowie eine lang anhaltende Wirkung aufweisen.

Die Erfindung betrifft auch ein Verfahren zur Herstellung der Verbindungen der Formel (I).

Das erfindungsgemäße Verfahren besteht darin, daß man ein 2-subst.-4-Amino-6,7-dimethoxy-chinazolin der allgemeinen Formel (II)

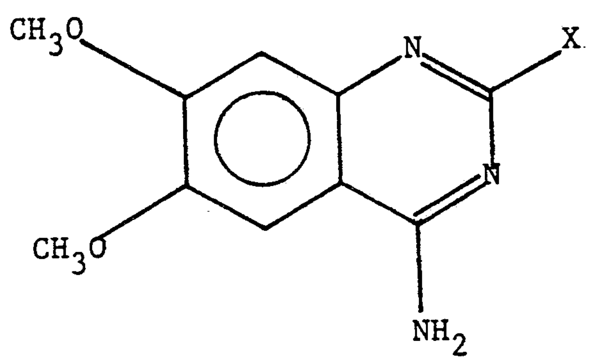

(II) ,

worin X eine als HX abspaltbare funktionelle Gruppe, wie z.B. ein Halogenatom, darstellt, vorzugsweise in einem inerten Lösungsmittel oder Lösungsmittelgemisch und gegebenenfalls in Anwesenheit eines Phasentransferkatalysators mit einem Amin der allgemeinen Formel (III)

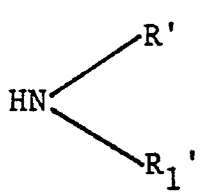

(III) ,

worin R' und $R_1$' entweder die oben für R und $R_1$ genannte Bedeutung haben, umsetzt, oder worin R' und $R_1$' einen unsubstituierten Piperazinring bilden, der nach erfolgter Umsetzung mit (II) durch weitere Reaktion

mit einem Lacton in einen 4-(Hydroxyacyl)-1-piperazinyl-Rest übergeführt wird, und die erhaltenen Derivate der allgemeinen Formel (I) gewünschtenfalls mit physiologisch verträglichen Säuren in Salze überführt.

Die als Ausgangsprodukte zum erfindungsgemäßen Verfahren verwendeten Chinazoline der allgemeinen Formel (II) sind bekannt (Journ.Chem.Soc. 1948, 1759; Journ.Med.Chem. 20, 148 (1977)) oder können in an sich bekannter Weise hergestellt werden. Das gilt auch für die Amin-Komponenten der allgemeinen Formel (III),
Die erfindungsgemäßen Verbindungen können entweder allein oder zusammen mit pharmazeutisch verwendbarem Trägermaterial gegen Bluthochdruck gegeben werden.

Die galenische Verarbeitung zu Tabletten, Kapseln, Lösungen, Suspensionen oder Suppositorien erfolgt in der dazu jeweils geeigneten Weise unter Verwendung der dem Fachmann an sich bekannten Hilfsstoffe.

Zur Herabsetzung des Blutdruckes von Hypertonikern wird die Dosierung dem vorgegebenen Überdruck angepaßt und mit relativ geringen Dosen begonnen. Im allgemeinen wird man pro Applikation mit 1 bis 100 mg Wirkstoff auskommen.

Durch die folgenden Beispiele soll die Erfindung näher erläutert, aber nicht auf diese beschränkt werden. Temperaturangaben beziehen sich jeweils auf Celsiusgrade.

**Beispiel 1 :**

Eine Mischung von 2 g 2-Chlor-4-amino-6,7-dimethoxy-chinazolin und 2 g Triäthylbenzylammoniumchlorid wird unter Rühren in 20 ml Amylalkohol zum Sieden erhitzt und im Verlauf von 5 h tropfenweise mit 3 ml N-(3-Aminopropyl)-2-pyrrolidinon versetzt. Nach 15 h Reaktionsdauer werden weitere 1,5 ml der Pyrrolidinon-Base zugetropft und erhitzt, bis eine Probe am Dünnschicht-Chromatogramm (Fertigplatte: Kieselgel 60 F 254, Laufmittel: Äthylacetat/Methanol/2n-Ammoniak = 25 : 8 : 3) vollständige Umsetzung anzeigt.

Die am Rotationsverdampfer eingeengte Mischung wird filtriert und der Niederschlag mit Isopropanol gewaschen (1,4 g Rohprodukt). Das Filtrat wird zur Trockene eingedampft, der Rückstand in Wasser und Chloroform aufgenommen, die Chloroform-Phase mit 1n HCl ausgeschüttelt, die salzsaure Phase neutralisiert und wieder mit Chloroform extrahiert. Nach Trocknung dieser Chloroformlösung mit $Na_2SO_4$ sicc. erhält man einen Eindampfrückstand, der beim Anreiben mit Isopropanol kristallisiert. Dieses Rohprodukt wird zusammen mit dem oben erhaltenen in Methanol gelöst und durch Zusatz von alkohol. HCl in das Hydrochlorid von 2-[3-(2-Pyrrolidinon-1-yl)-propyl-amino]-4-amino-6,7-dimethoxy-chinazolin übergeführt, welches bei 268 - 271° (Zers.) schmilzt.

**Beispiel 2 :**

Eine Mischung von 5 g 2-Chlor-4-amino-6,7-dimethoxy-chinazolin, 30 g N-Methyl-homoveratrylamin und 1 g Triäthylbenzylammoniumchlorid wird unter Rühren 3 h auf 110° erhitzt; danach destilliert man überschüssiges N-Methyl-homoveratrylamin im Vakuum ab. Aus der Lösung des Eindampfrückstandes in Aceton bildet sich beim Stehen in der Kälte ein Niederschlag. Dieser wird aus Methanol nach Filtration mit Aktivkohle umkristallisiert. Das erhaltene N-Methyl-N-(4-amino-6,7-dimethoxychinazolin-2-yl)-homoveratrylamin-Hydrochlorid schmilzt unter Zersetzung bei 165 - 270°.

**Beispiel 3 :**

Eine Suspension von 4,9 g 2-Chlor-4-amino-6,7-dimethoxy-chinazolin in einer Lösung von 4,87 g 4-(2-Keto-1-benzimidazolinyl)-piperidin in 150 ml n-Butanol wird unter Rückfluß gerührt, bis eine klare Lösung entstanden ist, aus der wieder ein Niederschlag ausfällt und eine Probe am Dünnschicht-Chromatogramm (Fertigplatte: Kieselgel 60 F 254, Laufmittel: Äthylacetat/Methanol/2n-Ammoniak = 25 : 8 : 3) vollständige Umsetzung anzeigt.

Die am Rotationsverdampfer eingeengte Mischung wird mit Aceton versetzt und das ausgefallene Hydrochlorid von 2-[4-(2-Keto-1-benzimidazolinyl)-piperidino]-4-amino-6,7-dimethoxy-chinazolin aus Methanol unter Filtration mit Aktivkohle umkristallisiert. Es schmilzt nach einer bei 287° beginnenden Zersetzung bei 296 - 298°.

**Beispiel 4 :**

5 g 2-Piperazinyl-4-amino-6,7-dimethoxy-chinazolin werden in 15 g $\gamma$-Butyrolacton auf 100° erhitzt, bis eine Probe am Dünnschicht-Chromatogramm (vgl. Beispiel 1 und 3) vollständige Umsetzung anzeigt. Der

über Nacht unter Kühlung ausgefallene Niederschlag wird abgesaugt und aus Äthanol umkristallisiert. Das erhaltene 1-(4-Amino-6,7-dimethoxychinazolin-2-yl)-4-(4-hydroxybutyryl)-piperazin zeigt bei 135 - 140° eine Kristallumwandlung und schmilzt bei 241 - 243°.

**Beispiel 5 :**

Zu einer bei 120° gerührten Schmelze von 15 g Piperazin tropft man langsam 6 g $\gamma$-Butyrolacton. Das Ende der Reaktion ist am Dünnschicht-Chromatogramm (vgl. Beispiel 1) nach Bedampfen mit Jod zu erkennen. Man dampft überschüssiges Piperazin im Vakuum ab und destilliert das zurückbleibende gelbe Öl bei 48 - 50°/399 Pa. Das 1-(4-Hydroxybutyryl)-piperazin kristallisiert beim Stehen und wird aus Aceton/Essigester umgefällt. Fp.: 99 - 101°.

1,8 g dieses Produktes werden zusammen mit 2,4 g 2-Chlor-4-amino-6,7-dimethoxy-chinazolin in 20 ml n-Butanol bei 100° gerührt. Nach 4 h wird die Mischung gekühlt und der entstandene Niederschlag abgesaugt. Nach Umkristallisation aus Methanol/Essigester erhält man das in Beispiel 4 beschriebene Produkt als Hydrochlorid, Fp. 210 - 215°.

**Beispiel 6 :**

5 g 2-Piperazinyl-4-amino-6,7-dimethoxy-chinazolin werden in 15 g $\beta$-Butyrolacton 3 h auf 100° erhitzt. Man verdünnt das erkaltete Reaktionsgemisch mit Essigester und saugt den über Nacht unter Kühlung ausgefallenen Niederschlag ab. Die Mutterlauge wird mit 2n HCl ausgeschüttelt, der saure Extrakt mit 50 %iger NaOH alkalisch gemacht und mehrmals mit Chloroform extrahiert. Der nach dem Trocknen der Chloroformlösung und deren Filtration mit Aktivkohle verbleibende Eindampfrückstand wird zusammen mit dem bereits erhaltenen Niederschlag aus Essigester umkristallisiert. Das 1-(4-Amino-6,7-dimethoxy-chinazolin-2-yl)-4-(3-hydroxy-butyryl)-piperazin schmilzt bei 220 - 222°.

**Beispiel 7 :**

Zu einer bei 120° gerührten Schmelze von 25 g Piperazin tropft man langsam 15 ml $\beta$-Butyrolacton. Dann dampft man überschüssiges Piperazin im Vakuum ab und destilliert den Rückstand bei ca. 60°/532 Pa. Das erhaltene Öl besteht aus 1-(3-Hydroxybutyryl)-piperazin und hat einen Brechungsindex $n_D^{25}$ von 1,5586.

1,8 g dieses Produkts werden mit 2 g 2-Chlor-4-amino-6,7-dimethoxy-chinazolin in 20 ml n-Butanol bei 100° gerührt. Nach 6 h wird die Mischung gekühlt und der entstandene Niederschlag abgesaugt. Es handelt sich um das Hydrochlorid des im Beispiel 6 erhaltenen Produktes. Fp. (Zers.): 281°.

Nach den beschriebenen Verfahren wurden weitere, in folgender Tabelle dargestellte Verbindungen erhalten.

| Derivate der allgemeinen Formel (I) | | |
|---|---|---|
| 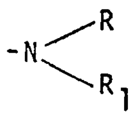 | Base/Salz | Fp °C |
| 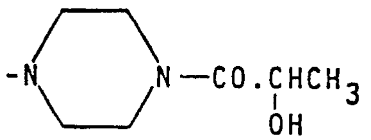 | Hydrochlorid | 230 - 232 |
| 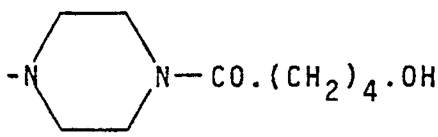 | Base | 208 - 210 |
| 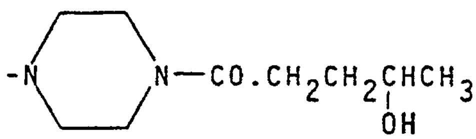 | Base | 233 - 235 |
| 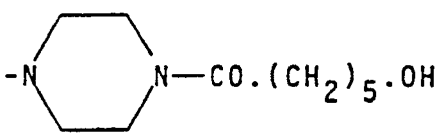 | Base | 162 - 164 |
| 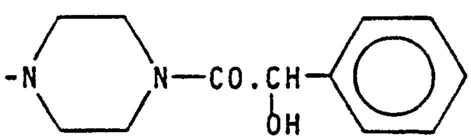 | Hydrochlorid | 260 - 262 |

Das als Zwischenprodukt verwendete Mandelsäure-piperazid (N-(2-Hydroxy-2-phenyl-acetyl)-piperazin) wurde durch Erhitzen von Mandelsäuremethylester mit überschüssigem Piperazin auf 120° erhalten. Nach Abdampfen von unumgesetztem Piperazin verbleibt ein Öl, das beim Anreiben mit Isopropyläther kristallisiert, Fp. 192°.

**Patentansprüche**

**Patentansprüche für folgende Vertragsstaaten : BE, CH, DE, FR, GB, IT, LI, LU, NL, SE**

**1.** Neue Chinazolin-Derivate der allgemeinen Formel (I)

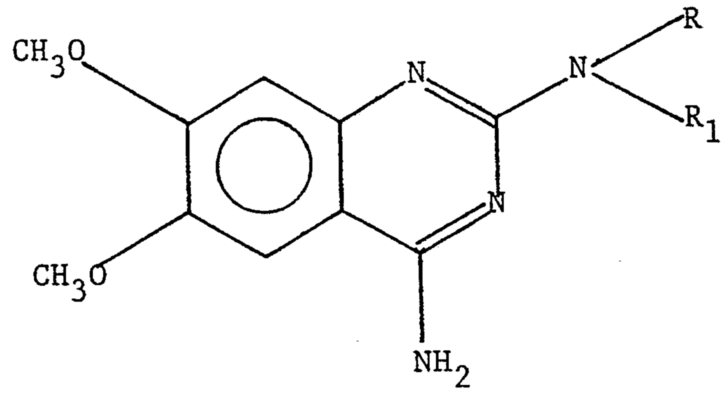

(I) ,

worin entweder R Wasserstoff und $R_1$ einen 3-(2-Pyrrolidinon-1-yl)-propyl-Rest darstellt oder worin R Methyl und $R_1$ einen 2-(Dimethoxyphenyl)-äthyl-Rest bedeutet oder worin R zusammen mit $R_1$ einen Piperidinring bildet, in dessen 4-Stellung ein 2-Keto-1-benzimidazolinyl-Rest gebunden ist oder worin R zusammen mit $R_1$ einen Piperazinring bildet, in dessen 4-Stellung ein Hydroxyacylrest mit höchstens 8 Kohlenstoffatomen gebunden ist.

2. Verfahren zur Herstellung der Chinazolin-Derivate der Formel (I) gemäß Anspruch 1, **dadurch gekennzeichnet,** daß man ein 2-subst. 4-Amino-6,7-dimethoxy-chinazolin der allgemeinen Formel (II)

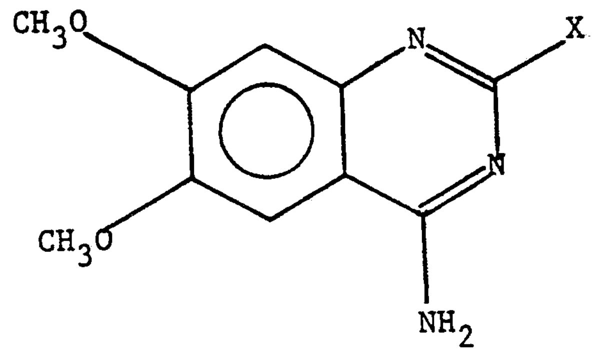

(II) ,

worin X eine als HX abspaltbare funktionelle Gruppe, wie z.B. ein Halogenatom, darstellt, vorzugsweise in einem inerten Lösungsmittel oder Lösungsmittelgemisch und gegebenenfalls in Anwesenheit eines Phasentransferkatalysators mit einem Amin der allgemeinen Formel (III)

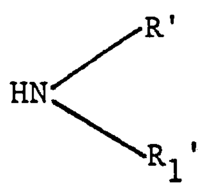

(III) ,

worin R′ und $R_1'$ entweder die oben für R und $R_1$ genannte Bedeutung haben, umsetzt, oder worin R′ und $R_1'$ einen unsubstituierten Piperazinring bilden, der nach erfolgter Umsetzung mit (II) durch weitere Reaktion mit einem Lacton in einen 4-(Hydroxyacyl)-1-piperazinyl-Rest übergeführt wird, und die erhaltenen Derivate der allgemeinen Formel (I) gewünschtenfalls mit physiologisch verträglichen Säuren in Salze überführt.

3. Pharmazeutische Zusammensetzung enthaltend eine den Blutdruck senkende Menge eines Chinazolin-Derivats der Formel (I) gemäß Anspruch 1.

4. Verwendung von Chinazolin-Derivaten der allgemeinen Formel (I) gemäß Anspruch 1, allein oder in

Kombination mit anderen geeigneten Wirkstoffen und unter Verwendung von an sich bekannten Hilfsstoffen für die galenische Verarbeitung, zur Senkung des Bluthochdrucks.

**Patentansprüche für folgenden Vertragsstaat : GR**

1. Neue Chinazolin-Derivate der allgemeinen Formel (I)

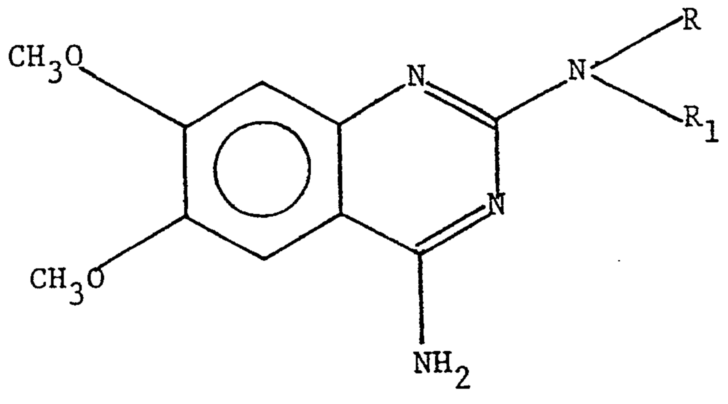

(I) ,

worin entweder R Wasserstoff und $R_1$ einen 3-(2-Pyrrolidinon-1-yl)-propyl-Rest darstellt oder worin R Methyl und $R_1$ einen 2-(Dimethoxyphenyl)-äthyl-Rest bedeutet oder worin R zusammen mit $R_1$ einen Piperidinring bildet, in dessen 4-Stellung ein 2-Keto-1-benzimidazolinyl-Rest gebunden ist oder worin R zusammen mit $R_1$ einen Piperazinring bildet, in dessen 4-Stellung ein Hydroxyacylrest mit höchstens 8 Kohlenstoffatomen gebunden ist.

2. Verfahren zur Herstellung der Chinazolin-Derivate der Formel (I) gemäß Anspruch 1, **dadurch gekennzeichnet,** daß man ein 2-subst. 4-Amino-6,7-dimethoxy-chinazolin der allgemeinen Formel (II)

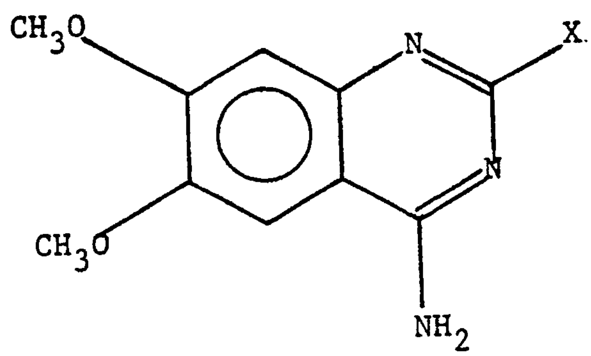

(II) ,

worin X eine als HX abspaltbare funktionelle Gruppe, wie z.B. ein Halogenatom, darstellt, vorzugsweise in einem inerten Lösungsmittel oder Lösungsmittelgemisch und gegebenenfalls in Anwesenheit eines Phasentransferkatalysators mit einem Amin der allgemeinen Formel (III)

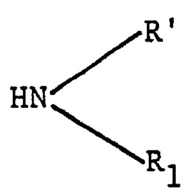

(III) ,

worin R′ und $R_1$′ entweder die oben für R und $R_1$ genannte Bedeutung haben, umsetzt, oder worin R′ und $R_1$′ einen unsubstituierten Piperazinring bilden, der nach erfolgter Umsetzung mit (II) durch weitere

Reaktion mit einem Lacton in einen 4-(Hydroxyacyl)-1-piperazinyl-Rest übergeführt wird, und die erhaltenen Derivate der allgemeinen Formel (I) gewünschtenfalls mit physiologisch verträglichen Säuren in Salze überführt.

3. Verwendung von Chinazolin-Derivaten der allgemeinen Formel (I) gemäß Anspruch 1, allein oder in Kombination mit anderen geeigneten Wirkstoffen und unter Verwendung von an sich bekannten Hilfsstoffen für die galenische Verarbeitung, zur Senkung des Bluthochdrucks.

**Patentanspruch für folgenden Vertragsstaat : ES**

1. Verfahren zur Herstellung der neuen Chinazolin-Derivate der allgemeinen Formel (I)

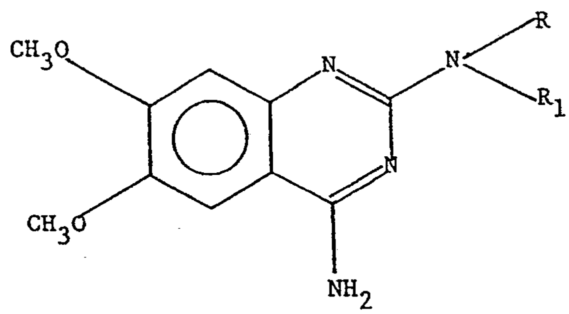

(I) ,

worin entweder R Wasserstoff und $R_1$ einen 3-(2-Pyrrolidinon-1-yl)-propyl-Rest darstellt oder worin R Methyl und $R_1$ einen 2-(Dimethoxyphenyl)-äthyl-Rest bedeutet oder worin R zusammen mit $R_1$ einen Piperidinring bildet, in dessen 4-Stellung ein 2-Keto-1-benzimidazolinyl-Rest gebunden ist oder worin R zusammen mit $R_1$ einen Piperazinring bildet, in dessen 4-Stellung ein Hydroxyacylrest mit höchstens 8 Kohlenstoffatomen gebunden ist, dadurch gekennzeichnet, daß man ein 2-subst.4-Amino-6,7-dimethoxy-chinazolin der allgemeinen Formel (II)

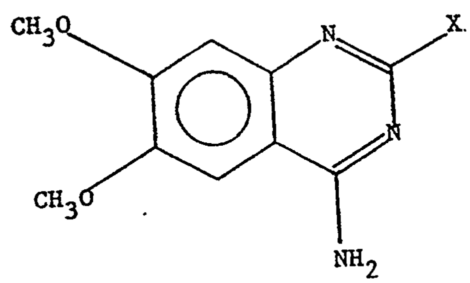

(II) ,

worin X eine als HX abspaltbare funktionelle Gruppe, wie z.B. ein Halogenatom, darstellt, vorzugsweise in einem inerten Lösungsmittel oder Lösungsmittelgemisch und gegebenenfalls in Anwesenheit eines Phasentransferkatalysators mit einem Amin der allgemeinen Formel (III)

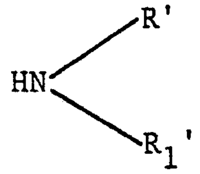

(III) ,

worin R′ und $R_1$′ entweder die oben für R und $R_1$ genannte Bedeutung haben, umsetzt, oder worin R′ und $R_1$′ einen unsubstituierten Piperazinring bilden, der nach erfolgter Umsetzung mit (II) durch weitere Reaktion mit einem Lacton in einen 4-(Hydroxyacyl)-1-piperazinyl-Rest übergeführt wird, und die erhaltenen Derivate der allgemeinen Formel (I) gewünschtenfalls mit physiologisch verträglichen Säuren in Salze überführt.

**Claims**

**Claims for the following Contracting States : BE, CH, DE, FR, GB, IT, LI, LU, NL, SE**

1. New quinazoline derivatives of general formula (I)

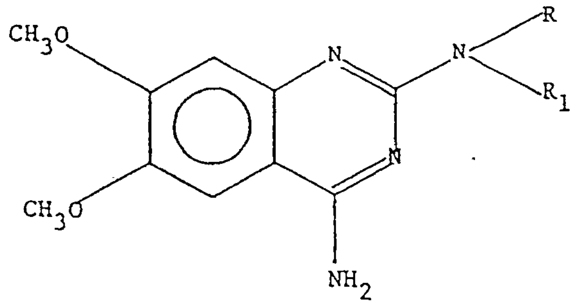

(I) ,

in which either R represents hydrogen and $R_1$ a 3-(2-pyrrolidinone-1-yl)propyl group or in which R stands for methyl and $R_1$ for a 2-(dimethoxyphenyl)ethyl group or in which R and $R_1$ together form a piperidine ring at whose 4-position a 2-keto-1-benzimidazolinyl group is bonded or in which R and $R_1$ together form a piperazine ring at whose 4-position a hydroxyacyl group with not more than 8 carbon atoms is bonded.

2. Process for the production of quinazoline derivatives of formula (I) according to Claim 1, characterized in that a 2-substituted 4-amino-6,7-dimethoxyquinazoline of general formula (II)

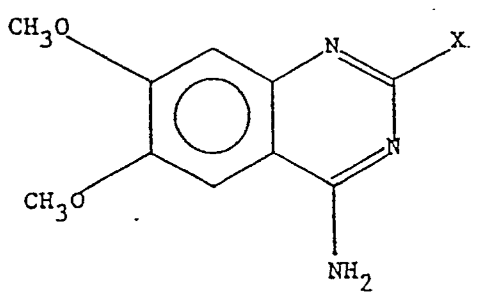

(II) ,

in which X represents a functional group which can be split off as HX, such as e.g. a halogen atom, is reacted, preferably in an inert solvent or solvent mixture and optionally in presence of a phase transfer catalyst, with an amine of general formula (III)

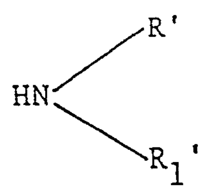

(III) ,

in which R' and $R_1$' either have the meaning mentioned above for R and $R_1$ or in which R' and $R_1$' form an unsubstituted piperazine ring which, after reaction has occurred with (II), is converted by further reaction with a lactone to a 4-(hydroxyacyl)-1-piperazinyl group, and the derivatives of general formula (I) obtained are, if desired, converted with physiologically compatible acids to salts.

3. Pharmaceutical composition containing a blood pressure-reducing amount of a quinazoline derivative of formula (I) according to Claim 1.

4. Use of quinazoline derivatives of general formula (I) according to Claim 1, alone or in combination with other suitable active ingredients and using auxiliary agents known in themselves for the manufacture of medicaments, for reducing high blood pressure.

**Claims for the following Contracting State : GR**

1. New quinazoline derivatives of general formula (I)

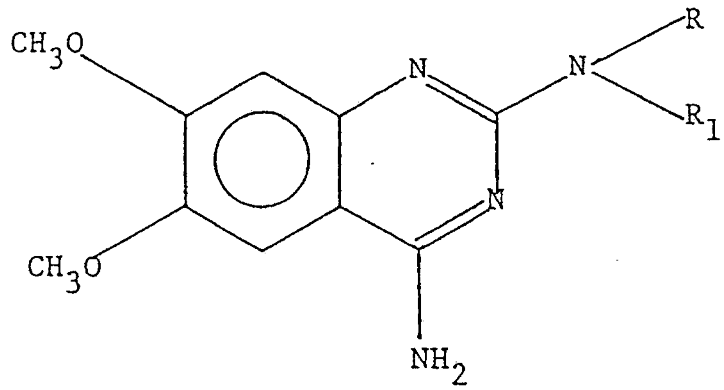

(I).,

in which either R represents hydrogen and $R_1$ a 3-(2-pyrrolidinone-1-yl)propyl group or in which R stands for methyl and $R_1$ for a 2-(dimethoxyphenyl)ethyl group or in which R and $R_1$ together form a piperidine ring at whose 4-position a 2-keto-1-benzimidazolinyl group is bonded or in which R and $R_1$ together form a piperazine ring at whose 4-position a hydroxyacyl group with not more than 8 carbon atoms is bonded.

2. Process for the production of quinazoline derivatives of formula (I) according to Claim 1, characterized in that a 2-substituted 4-amino-6,7-dimethoxyquinazoline of general formula (II)

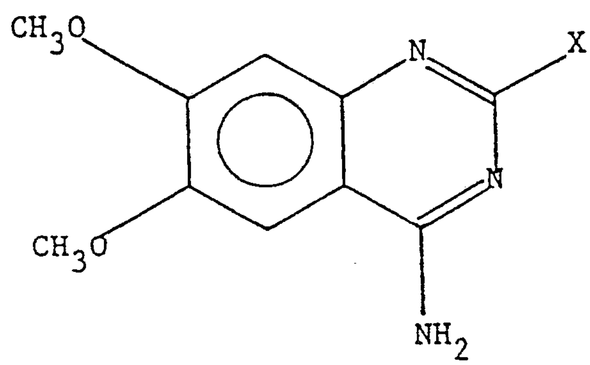

(II),

in which X represents a functional group which can be split off as HX, such as e.g. a halogen atom, is reacted, preferably in an inert solvent or solvent mixture and optionally in presence of a phase transfer catalyst, with an amine of general formula (III)

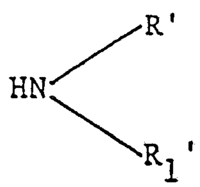

(III),

in which R' and $R_1$' either have the meaning mentioned above for R and $R_1$ or in which R' and $R_1$' form an unsubstituted piperazine ring which, after reaction has occurred with (II), is converted by further reaction with a lactone to a 4-(hydroxyacyl)-1-piperazinyl group, and the derivatives of general formula (I) obtained are, if desired, converted with physiologically compatible acids to salts.

3. Use of quinazoline derivatives of general formula (I) according to Claim 1, alone or in combination with

other suitable active ingredients and using auxiliary agents known in themselves for the manufacture of medicaments, for reducing high blood pressure.

**Claim for the following Contracting State : ES**

1. Process for the production of new quinazoline derivatives of general formula (I)

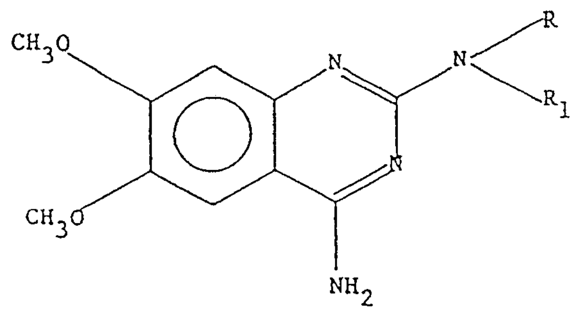

(I) ,

in which either R represents hydrogen and $R_1$ a 3-(2-pyrrolidinone-1-yl)propyl group or in which R stands for methyl and $R_1$ for a 2-(dimethoxyphenyl)ethyl group or in which R and $R_1$ together form a piperidine ring at whose 4-position a 2-keto-1-benzimidazolinyl group is bonded or in which R and $R_1$ together form a piperazine ring at whose 4-position a hydroxyacyl group with not more than 8 carbon atoms is bonded, characterized in that a 2-substituted 4-amino-6,7-dimethoxyquinazoline of general formula (II)

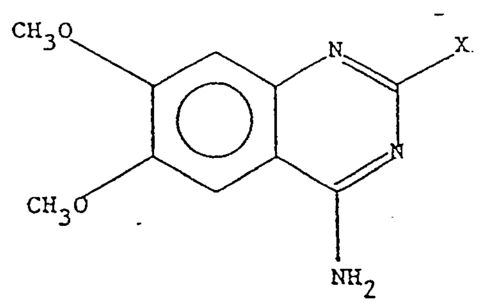

(II) ,

in which X represents a functional group which can be split off as HX, such as e.g. a halogen atom, is reacted, preferably in an inert solvent or solvent mixture and optionally in presence of a phase transfer catalyst, with an amine of general formula (III)

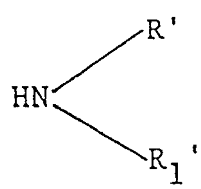

(III) ,

in which R' and $R_1$' either have the meaning mentioned above for R and $R_1$ or in which R' and $R_1$' form an unsubstituted piperazine ring which, after reaction has occurred with (II), is converted by further reaction with a lactone to a 4-(hydroxyacyl)-1-piperazinyl group, and the derivatives of general formula (I) obtained are, if desired, converted with physiologically compatible acids to salts.

**Revendications**

**Revendications pour les Etats contractants suivants : BE, CH, DE, FR, GB, IT, LI, LU, NL, SE**

1. Nouveaux dérivés de quinazoline de formule générale (I)

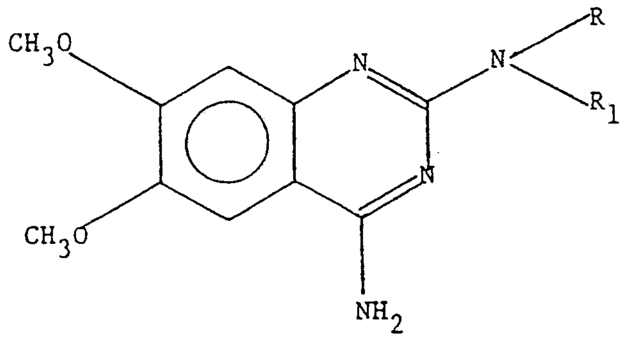

(I)

dans laquelle R représente un atome d'hydrogène quand $R_1$ représente un reste 3-(2-pyrrolidinon-1-yl)-propyle, ou bien R représente un groupe méthyle quand $R_1$ représente un reste 2-(diméthoxyphényl)-éthyle, ou encore R et $R_1$ forment ensemble un cycle pipéridine dans lequel la position 4 est occupée par un reste 2-céto-1-benzimidazolinyle, ou R et $R_1$ forment ensemble un cycle pipérazine dans lequel la position 4 est occupée par un reste hydroxyacyle comportant au plus 8 atomes de carbone.

**2.** Procédé de préparation des dérivés de quinazoline de formule générale (I) selon la revendication 1, caractérisé en ce que l'on fait réagir une 4-amino-6,7-diméthoxyquinazoline susbtituée en position 2, de formule générale (II)

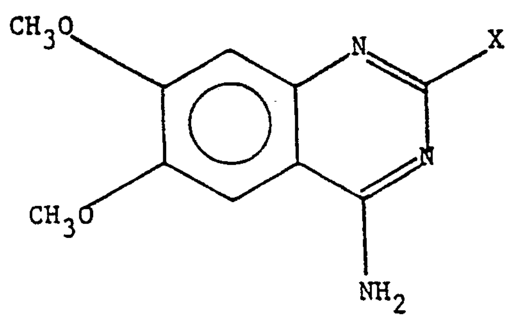

(II)

dans laquelle X représente un groupe fonctionnel séparable sous forme de HX, tel que p.ex. un atome d'halogène, de préférence dans un solvant ou un mélange de solvants inerte et éventuellement en présence d'un catalyseur de transfert de phase, avec une amine de formule générale (III)

$R'-NH-R_1'$ (III) ,

dans laquelle R' et $R_1'$ ont les définitions données ci-dessus, ou bien dans laquelle R' et $R_1'$ forment ensemble un cycle pipérazine non substitué qui, après réaction avec (II), est transformé en un reste 4-(hydroxyacyl)-1-pipérazinyle par réaction ultérieure avec une lactone, et les dérivés de formule générale (I) obtenus sont convertis, si on le souhaite, en sels avec des acides physiologiquement acceptables.

**3.** Composition pharmaceutique contenant une quantité hypotensive d'un dérivé de quinazoline de formule (I) selon la revendication 1.

**4.** Utilisation de dérivés de quinazoline de formule générale (I) selon la revendication 1, seuls ou associés à d'autres principes actifs appropriés, et en présence d'adjuvants connus en soi pour la mise en forme galénique, pour abaisser l'hypertension sanguine.

**Revendications pour l'Etat contractant suivant : GR**

**1.** Nouveaux dérivés de quinazoline de formule générale (I)

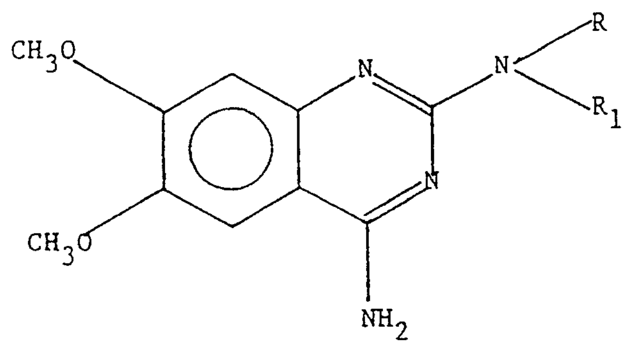

(I)

dans laquelle R représente un atome d'hydrogène quand $R_1$ représente un reste 3-(2-pyrrolidinon-1-yl)-propyle, ou bien R représente un groupe méthyle quand $R_1$ représente un reste 2-(diméthoxyphényl)-éthyle, ou encore R et $R_1$ forment ensemble un cycle pipéridine dans lequel la position 4 est occupée par un reste 2-céto-1-benzimidazolinyle, ou R et $R_1$ forment ensemble un cycle pipérazine dans lequel la position 4 est occupée par un reste hydroxyacyle comportant au plus 8 atomes de carbone.

**2.** Procédé de préparation des dérivés de quinazoline de formule générale (I) selon la revendication 1, caractérisé en ce que l'on fait réagir une 4-amino-6,7-diméthoxyquinazoline susbtituée en position 2, de formule générale (II)

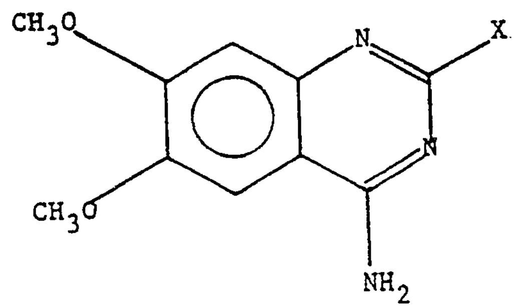

(II)

dans laquelle X représente un groupe fonctionnel séparable sous forme de HX, tel que p.ex. un atome d'halogène, de préférence dans un solvant ou un mélange de solvants inerte et éventuellement en présence d'un catalyseur de transfert de phase, avec une amine de formule générale (III)

$R'$-NH-$R_1'$ (III) ,

dans laquelle R' et $R_1'$ ont les définitions données ci-dessus, ou bien dans laquelle R' et $R_1'$ forment ensemble un cycle pipérazine non substitué qui, après réaction avec (II), est transformé en un reste 4-(hydroxyacyl)-1-pipérazinyle par réaction ultérieure avec une lactone, et les dérivés de formule générale (I) obtenus sont convertis, si on le souhaite, en sels avec des acides physiologiquement acceptables.

**3.** Utilisation de dérivés de quinazoline de formule générale (I) selon la revendication 1, seuls ou associés à d'autres principes actifs appropriés, et en présence d'adjuvants connus en soi pour la mise en forme galénique, pour abaisser l'hypertension sanguine.

**Revendication pour l'Etat contractant suivant : ES**

**1.** Procédé de préparation des nouveaux dérivés de quinazoline de formule générale (I)

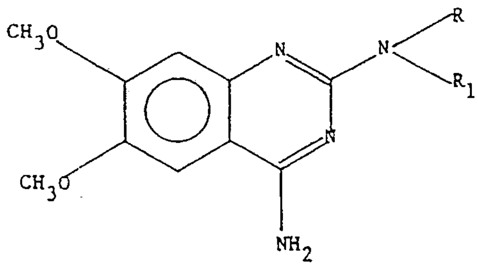

(I)

dans laquelle R représente un atome d'hydrogène quand $R_1$ représente un reste 3-(2-pyrrolidinon-1-yl)-propyle, ou bien R représente un groupe méthyle quand $R_1$ représente un reste 2-(diméthoxyphényl)-éthyle, ou encore R et $R_1$ forment ensemble un cycle pipéridine dans lequel la position 4 est occupée par un reste 2-céto-1-benzimidazolinyle, ou R et $R_1$ forment ensemble un cycle pipérazine dans lequel la position 4 est occupée par un reste hydroxyacyle comportant au plus 8 atomes de carbone, caractérisé en ce que l'on fait réagir une 4-amino-6,7-diméthoxyquinazoline susbtituée en position 2, de formule générale (II)

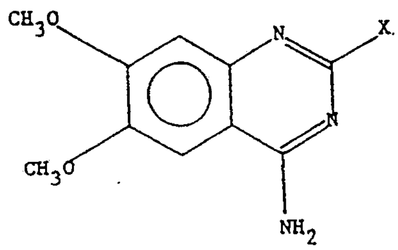

(II)

dans laquelle X représente un groupe fonctionnel séparable sous forme de HX, tel que p.ex. un atome d'halogène, de préférence dans un solvant ou un mélange de solvants inerte et éventuellement en présence d'un catalyseur de transfert de phase, avec une amine de formule générale (III)

$R'-NH-R_1'$ (III) ,

dans laquelle R' et $R_1'$ ont les définitions données ci-dessus, ou bien dans laquelle R' et $R_1'$ forment ensemble un cycle pipérazine non substitué qui, après réaction avec (II), est transformé en un reste 4-(hydroxyacyl)-1-pipérazinyle par réaction ultérieure avec une lactone, et les dérivés de formule générale (I) obtenus sont convertis, si on le souhaite, en sels avec des acides physiologiquement acceptables.